## Europäisches Patentamt
## European Patent Office
## Office européen des brevets

(19)

(11) Veröffentlichungsnummer: **0 051 148**
**B1**

(12)

# EUROPÄISCHE PATENTSCHRIFT

(45) Veröffentlichungstag der Patentschrift:
08.08.84

(51) Int. Cl.³: **C 07 C 69/003**, C 07 C 67/08

(21) Anmeldenummer: **81107676.9**

(22) Anmeldetag: **26.09.81**

(54) **Wollwachssubstitute.**

(30) Priorität: **31.10.80 DE 3041073**

(43) Veröffentlichungstag der Anmeldung:
**12.05.82 Patentblatt 82/19**

(45) Bekanntmachung des Hinweises auf die Patenterteilung:
**08.08.84 Patentblatt 84/32**

(84) Benannte Vertragsstaaten:
**AT BE CH DE FR GB IT LI LU NL SE**

(56) Entgegenhaltungen:
**EP - A - 0 014 308**
**DE - A - 2 605 329**

(73) Patentinhaber: **DYNAMIT NOBEL AKTIENGESELLSCHAFT, Postfach 1209, D-5210 Troisdorf, Bez. Köln (DE)**

(72) Erfinder: **Naskar, Sasanka Sekhar, Parkweg 63, D-5810 Witten (DE)**
Erfinder: **Hülsmann, Hans Leo, Dr., Appendahl 29, D-5802 Wetter 4 (DE)**
Erfinder: **Pass, Reinhard, Brink 10, D-5810 Witten (DE)**

## Beschreibung

Die Erfindung betrifft ein Wollwachssubstitut auf Basis einheitlicher Estergemische aus Veresterungsprodukten von Glycerin-Polyglycerin-Gemischen eines mittleren Molekulargewichts zwischen 140 und 210 mit ausgewählten Gemischen gesättigter aliphatischer Monocarbonsäuren und Dicarbonsäuren, wobei die Estergemische Hydroxylzahlen von 20 bis 100, Säurezahlen und Jodzahlen unter 5 haben sowie ein Verfahren zu deren Herstellung.

Ziel der Erfindung ist es, ein Wollwachssubstitut auf Basis eines einheitlichen Estergemisches zu gewinnen, dessen Hautauftragsverhalten dem des natürlichen Wollwachses entspricht und auch technisch zugänglichen, physiologisch einwandfreien Rohstoffen nach einem wenig aufwendigen Verfahren erhältlich ist. Die erfindungsgemäß hergestellten Estergemische finden besondere Anwendung im kosmetischen und pharmazeutischen Bereich, wie auch im technischen Bereich als Schmiermittelhilfsstoff und als Weichmacher für Kleber und Harze.

Wollwachs, auch Lanolin genannt, ist ein relativ teures Naturprodukt. Die Gewinnung und Reinigung ist kostspielig. Entsprechend seiner geographischen Herkunft bzw. den Fütterungsbedingungen der Schafe kann Wollwachs in seiner Zusammensetzung schwanken. Nachteilig ist ein häufiger Gehalt von Pestiziden.

Verunreinigungen durch Detergentien, Bleichmittel, Aldehyde bzw. Ketone, entstanden durch oxidierende Behandlung des Wollwachses, vermindern die Qualität des natürlichen Produktes. In den letzten Jahren ist Wollwachs als Verursacher von allergischen Reaktionen bei empfindlicher Haut in Verdacht geraten.

Natürliches Wollwachs ist eine Substanz mit komplizierter Zusammensetzung. Im wesentlichen ist es ein Gemisch von ca. 95% Estern höherer Fettsäuren, wie Hydroxyfettsäuren, gerad- und verzweigtkettigen Monocarbonsäuren mit 10 bis 31 C-Atomen und Cholesterin, Dihydroxycholesterin, Isocholesterin, aliphatischen höheren Alkoholen mit 16 bis 30 C-Atomen, höhermolekularen Diolen, neben geringen Mengen an freien Paraffinkohlenwasserstoffen, freien Säuren und freien Alkoholen.

Wollwachs wird durch Extraktions- oder Waschprozesse der Schafswolle gewonnen. Das Rohprodukt wird stufenweise gereinigt, gebleicht und desodorisiert und bildet dann eine hellgelbe, schwach riechende, zähe Substanz. Gereinigtes Wollwachs besitzt ein hervorragendes Hautauftragsverhalten und wird seit jeher in verschiedenen pharmazeutischen und kosmetischen Rezepturen geschätzt.

Gegenstand der Erfindung ist ein Wollwachssubstitut auf Basis einheitlicher Estergemische, gekennzeichnet durch Veresterungsprodukte von 1 Mol Glycerin-Polyglycerin-Gemisch eines mittleren Molekulargewichts zwischen 140 und 210 mit

a) 0,5 bis 1,1 Mol einer gesättigten aliphatischen Monocarbonsäure mit 6 bis 10 C-Atomen oder deren Gemischen,

b) 0,5 bis 1,1 Mol einer gesättigten aliphatischen Monocarbonsäure mit 16 bis 22 C-Atomen oder deren Gemischen,

c) 0,0 bis 0,6 Mol einer aliphatischen gesättigten verzweigtkettigen Monocarbonsäure mit 16 bis 18 C-Atomen oder deren Gemischen,

d) 0,0 bis 0,5 Mol einer Hydroxylgruppen enthaltenden gesättigten Monocarbonsäure mit 16 bis 20 C-Atomen oder deren Gemischen und

e) 0,5 bis 1,0 Mol gesättigten aliphatischen Dicarbonsäure mit 4 bis 10 C-Atomen oder deren Gemischen mit einer Hydroxylzahl von 20 bis 100, einer Säurezahl und Jodzahl unter 5.

Die erfindungsgemäßen Wollwachssubstitute besitzen eine sehr gute Wasseraufnahmefähigkeit und Emulgierbarkeit. Zahlreiche hergestellte Wollwachssubstitute, insbesondere solche des Vorzugsbereiches, übertreffen die Wasseraufnahme guter Wollwachse mit Wasserzahlen zwischen 100 und 260.

Die Emulgierbarkeit und die Stabilität hergestellter Emulsionen ist mit dem Wollwachs vergleichbar und als gut zu beurteilen.

Die Produkte besitzen eine zähe fädenziehende Konsistenz, die der für Wollwachs charakteristischen »Fädigkeit« entspricht.

Hautauftragsverhalten, d. h. filmbildende Eigenschaften auf der Haut, werden von dem entwickelten Wollwachssubstitut vergleichbar erreicht.

Die Klebrigkeit der neuen Produkte bleibt auch nach mehrmonatiger Lagerung erhalten. Verschiedene kosmetische Rezepturen auf Basis der erfindungsgemäß erhaltenen Substitute erreichen oder übertreffen auch nach längerer Lagerung Zubereitungen aus Wollwachs.

Weiterer Gegenstand der Erfindung ist ein Verfahren zur Herstellung von Wollwachssubstituten auf Basis einheitlicher Estergemische, welches dadurch gekennzeichnet ist, daß man je 1 Mol eines Glycerin-Polyglycerin-Gemisches eines mittleren Molekulargewichts zwischen 140 und 210 mit 0,5 bis 1,1 Mol einer gesättigten aliphatischen Monocarbonsäure mit 6 bis 10 C-Atomen oder deren Gemischen, 0,5 bis 1,1 Mol einer gesättigten aliphatischen Monocarbonsäure mit 16 bis 22 C-Atomen oder deren Gemischen, 0,0 bis 0,6 Mol einer aliphatischen, gesättigten, verzweigtkettigen Monocarbonsäure mit 16 bis 18 C-Atomen oder deren Gemischen und 0,0 bis 0,5 Mol einer Hydroxylgruppen enthaltenden gesättigten Monocarbonsäure mit 16 bis 20 C-Atomen oder deren Gemischen bei 200 bis 250° C, vorzugsweise unter Vakuum zu hydroxylhaltigen Partialestern verestert, bis eine Säurezahl unter 50, vorzugsweise unter 10, erreicht ist und anschließend mit 0,5 bis 1,0 Mol einer aliphatischen gesättigten Dicarbonsäure mit 4 bis 10 C-Atomen oder deren Gemischen bei 200 bis 250°C, vorzugsweise unter Vakuum, weiterverestert, bis eine Hydroxylzahl von 20 bis 100 und eine Säurezahl unter 5, vorzugsweise unter 1, erreicht ist und das Rohprodukt in üblicher Weise desodorisiert.

Das erfindungsgemäße Wollwachssubstitut besteht aus einem aliphatischen Estergemisch, dessen Zusammensetzung entsprechend den angegebenen Hydroxylzahlen stets eine niedrigere Summe der Säurereste als die Summe der Hydroxylreste des Glycerin-Polyglycerin-Gemisches aufweist. Entsprechend der Säurezahlen unter 5, vorzugsweise unter 1, wird eine vollständige Veresterung der Säuren ausgeführt. Die geringe Jodzahl unter 5, vorzugsweise unter 1, ist durch Verwendung gesättigter Säuren und der nur geringen Gehalte an ungesättigter Säuren gewährleistet.

Bevorzugt enthalten die Veresterungsprodukte je Mol Glycerin-Polyglycerin-Gemisch 0,7 bis 1,0 a) 0,7 bis 1,0 b), 0,05 bis 0,4 der verzweigten Monocarbonsäure c), 0,05 bis 0,3 der Hydroxycarbonsäure d), sowie 0,60 bis 0,9 Mol der Komponente e).

Es ist demnach bevorzugt, daß mindestens die genannten kleinen Anteile der verzweigten Monocarbonsäure c) und/oder der Hydroxycarbonsäure d) enthalten sind, die zu verbesserten Produkten für eine Anzahl von Verwendungen führen.

Wichtiger Bestandteil ist das Glycerin-Polyglycerin-Gemisch. Es enthält neben Glycerin durch Kondensation gebildete Anteile von Di-, Tri- und höher kondensiertem Glycerin.

Das mittlere Molekulargewicht des Polyglycerin-Glycerin-Gemisches liegt zwischen 140 und 210, bevorzugt zwischen 160 und 180 (gemessen durch Dampfdruck-Osmometrie). Die Viskosität liegt zwischen 300 und 500 mPa s, gemessen bei 65°C.

Glycerin-Polyglycerin-Gemische können durch Kondensation von Glycerin bei z. B. 240 bis 260°C und 300 bis 600 mbar mit z. B. Natriummethylat als Katalysator hergestellt werden. Die Komponenten a) und b) sind geradkettige Fettsäuren. Als Komponente a) werden Gemische bevorzugt, insbesondere die bei der Destillation von Cocosfettsäure anfallende Cocosvorlauffettsäure, die 0,3—3,0 $C_6$-, 50—65 $C_8$- und 30—50 Gew.-% $C_{10}$-Säure, 1,0—3,0 Gew.-% $C_{12}$-Säure, bevorzugt 0,4—1,3 $C_6$-, 52—64 $C_8$- und 32—46 Gew.-% $C_{10}$-Säure, 1,8—2,6 Gew.-% $C_{12}$-Säure enthält. Als Komponente b) sind Palmitin- und besonders Stearinsäure und deren Mischungen bevorzugt.

Als Komponente c) wird die sogenannte Isostearinsäure bevorzugt. Sie ist ein Gemisch zahlreicher isomerer, hauptsächlich methylverzweigter $C_{18}$-Fettsäuren mit niedrigen Jodzahlen und dadurch geringer Oxidationsneigung (H. Janistyn — Handbuch der Kosmetika und Riechstoffe, Band 1, S 533 — Hersteller: Unilever Emmery N. V., Condea Holland). Als Komponente d) sind Hydroxysäuren mit einer Hydroxylgruppe, besonders die 12-Hydroxystearinsäure, bevorzugt. Als Komponente e) sind Bernsteinsäure, Adipinsäure und Sebacinsäure bevorzugt.

Die neuen Wollwachssubstitute haben den Vorteil einheitlicher Estergemische, deren Eigenschaften der jeweiligen Verwendung angepaßt werden können.

Die Verwendung ist daher für alle die Zwecke auf kosmetischem, pharmazeutischem wie auf ggf. technischem Gebiet vorgesehen, in denen Wollwachs Verwendung findet.

Beispiel 1

In einem 2-Liter-Dreihalskolben, der mit Rührwerk, Wasserabscheider, Dephlegmator, Thermometer und Gaseinleitungsrohr versehen ist, wird eine Mischung von 186,8 g (1,10 Mol) Glycerin-Polyglycerin-Gemisch, mittleres Molekulargewicht 170, a) 107,8 g (0,691 Mol) Cocosvorlauffettsäure, (Zusammensetzung: 0,5 Gew.-% $C_6$-, 62,5 Gew.-% $C_8$-, 35,0 Gew.-% $C_{10}$- und 2,0 Gew.-% $C_{12}$-Säure), b) 257,8 g (0,906 Mol) Stearinsäure, c) 113,8 g (0,4 Mol) Isostearinsäure, d) 30,1 g (0,1 Mol) 12-Hydroxystearinsäure und 0,5 g Isopropyltitanat unter Rühren und gleichzeitigem Durchleiten von Inertgas während 2 Stunden bei 400 mbar bis 240°C erhitzt und das Reaktionswasser bis zu einer Säurezahl kleiner als 10 entfernt. Der erhaltene Kolbeninhalt wird auf 140°C abgekühlt. Nach Zugabe von e) 116,8 g (0,8 Mol) Adipinsäure wird unter Rühren und Inertgas auf 240°C aufgeheizt und das anfallende Reaktionswasser gleichzeitig abgenommen. Bei schrittweiser Verbesserung des Vakuums bis auf 5 mbar wird bis zu einer Säurezahl unter 2 verestert. Der erhaltene Rohester wird in üblicher Weise desodorisiert und filtriert.

Kennzahlen:
| | |
|---|---|
| Säurezahl | 0,5 |
| Verseifungszahl | 283 |
| Hydroxylzahl | 60 |

Beispiel 2

Analog Beispiel 1 werden 186,8 g (1,1 Mol) Glycerin-Polyglycerin-Gemisch, mittleres Molekulargewicht 170, mit a) 142,9 g (0,916 Mol) Cocosvorlauffettsäure, Zusammensetzung: 0,5 Gew.-% $C_6$-, 62,5 Gew.-% $C_8$-, 35,0 Gew.-% $C_{10}$- und 2,0 Gew.-% $C_{12}$-Säure, b) 222,3 g (0,781 Mol) Stearinsäure, c) 113,4 g (0,4 Mol) Isostearinsäure, d) 30,1 g (0,1 Mol) 12-Hydroxystearinsäure und e) 102,2 g (0,7 Mol) Adipinsäure verestert.

Kennzahlen:
| | |
|---|---|
| Säurezahl | 0,75 |
| Verseifungszahl | 278 |
| Hydroxylzahl | 58 |

### Beispiel 3

Analog Beispiel 1 werden 186,8 g (1,1 Mol) Glycerin-Polyglycerin-Gemisch, mittleres Molekulargewicht 170, mit a) 131,2 g (0,841 Mol) Cocosvorlauffettsäure, Zusammensetzung: 0,5 Gew.-% $C_6$-, 62,5 Gew.-% $C_8$-, 35,0 Gew.-% $C_{10}$- und 2,0 Gew.-% $C_{12}$-Säure, b) 273,5 g (0,961 Mol) Stearinsäure, c) 42,6 g (0,15 Mol) Isostearinsäure, d) 45,1 g (0,15 Mol) 12-Hydroxystearinsäure und e) einem Dicarbonsäuregemisch bestehend aus 35,4 g (0,3 Mol) Bernsteinsäure, 43,8 g (0,3 Mol) Adipinsäure und 30,3 g (0,15 Mol) Sebacinsäure verestert.

Kennzahlen:

| | |
|---|---|
| Säurezahl | 1,0 |
| Verseifungszahl | 278 |
| Hydroxylzahl | 71 |

### Beispiel 4 (Verwendung)

Beispiel der pharmazeutischen Grundlagenrezeptur einer Salbe, in der das natürliche Wollwachs durch das aus Beispiel 3 erhaltene synthetische Wollwachsersatzprodukt unter Beibehaltung gleicher Eigenschaften ausgetauscht werden kann:

| | |
|---|---|
| Erdnußöl | 15% |
| Paraffin liq. | 21% |
| Vaseline | 22% |
| Hartparaffin | 7% |
| Borax | 0,3% |
| Wollwachs oder Wollwachssubstitut | 5% |
| dest. Wasser, ad. | 100% |

**Patentansprüche für die Vertragsstaaten: BE, CH, DE, FR, GB, IT, LI, LU, NL, SE**

1. Wollwachssubstitute auf Basis einheitlicher Estergemische, gekennzeichnet durch Veresterungsprodukte von 1 Mol Glycerin-Polyglycerin-Gemisch eines mittleren Molekulargewichts zwischen 140 und 210 mit

a) 0,5 bis 1,1 Mol einer gesättigten aliphatischen Monocarbonsäure mit 6 bis 10 C-Atomen oder deren Gemischen,

b) 0,5 bis 1,1 Mol einer gesättigten aliphatischen Monocarbonsäure mit 16 bis 22 C-Atomen oder deren Gemischen,

c) 0,0 bis 0,6 Mol einer aliphatischen gesättigten verzweigtkettigen Monocarbonsäure mit 16 bis 18 C-Atomen oder deren Gemischen,

d) 0,0 bis 0,5 Mol einer Hydroxylgruppen enthaltenden gesättigten Monocarbonsäure mit 16 bis 20 C-Atomen oder deren Gemischen und

e) 0,5 bis 1,0 Mol gesättigten aliphatischen Dicarbonsäuren mit 4 bis 10 C-Atomen oder deren Gemischen mit einer Hydroxylzahl von 20 bis 100, einer Säurezahl und Jodzahl unter 5.

2. Verfahren zur Herstellung von Wollwachssubstituten auf Basis einheitlicher Estergemische, dadurch gekennzeichnet, daß man je 1 Mol eines Glycerin-Polyglycerin-Gemisches eines mittleren Molekulargewichts zwischen 140 und 210 mit 0,5 bis 1,1 Mol einer gesättigten aliphatischen Monocarbonsäure mit 6 bis 10 C-Atomen oder deren Gemischen, 0,5 bis 1,1 Mol einer gesättigten aliphatischen Monocarbonsäure mit 16 bis 22 C-Atomen oder deren Gemischen, 0,0 bis 0,6 Mol einer aliphatischen, gesättigten, verzweigtkettigen Monocarbonsäure mit 16 bis 18 C-Atomen oder deren Gemischen und 0,0 bis 0,5 Mol einer Hydroxylgruppen enthaltenden gesättigten Monocarborsäure mit 16 bis 20 C-Atomen oder deren Gemischen bei 200 bis 250° C, vorzugsweise unter Vakuum zu hydroxylhaltigen Partialestern verestert, bis eine Säurezahl unter 50, vorzugsweise unter 10, erreicht ist und anschließend mit 0,5 bis 1,0 Mol einer aliphatischen gesättigten Dicarbonsäure mit 4 bis 10 C-Atomen oder deren Gemischen bei 200 bis 250° C, vorzugsweise unter Vakuum, weiterverestert, bis eine Hydroxylzahl von 20 bis 100 und eine Säurezahl unter 5, vorzugsweise unter 1, erreicht ist und das Rohprodukt in üblicher Weise desodorisiert.

**Patentanspruch für den Vertragsstaat: AT**

1. Verfahren zur Herstellung vor Wollwachssubstituten auf Basis einheitlicher Estergemische, dadurch gekennzeichnet, daß man je 1 Mol eines Glycerin-Polyglycerin-Gemisches eines mittleren Molekulargewichts zwischen 140 und 210 mit 0,5 bis 1,1 Mol einer gesättigten aliphatischen Monocarbonsäure mit 6 bis 10 C-Atomen oder deren Gemischen, 0,5 bis 1,1 Mol einer gesättigten aliphatischen Monocarbonsäure mit 16 bis 22 C-Atomen oder deren Gemischen, 0,0 bis 0,6 Mol einer aliphatischen, gesättigten, verzweigtkettigen Monocarbonsäure mit 16 bis 18 C-Atomen oder deren Gemischen und 0,0 bis 0,5 Mol einer Hydroxylgruppen enthaltenden gesättigten Monocarbonsäure mit 16 bis 20 C-Atomen oder deren Gemischen bei 200 bis 250° C, vorzugsweise unter Vakuum zu hydroxylhaltigen Partialestern verestert, bis eine Säurezahl unter 50, vorzugsweise unter 10, erreicht ist und anschließend mit 0,5 bis 1,0 Mol einer aliphatischen gesättigten Dicarbonsäure mit 4 bis 10 C-Atomen oder deren Gemischen bei 200 bis 250° C, vorzugsweise unter Vakuum, weiterverestert, bis eine Hydroxylzahl von 20 bis 100 und eine Säurezahl unter 5, vorzugsweise unter 1, erreicht ist und das Rohprodukt in üblicher Weise desodorisiert.

## Claims for the designated states: BE, CH, DE, FR, GB, IT, LI, LU, NL, SE

1. Wool wax substitutes based on homogeneous ester mixtures, characterised by esterification products of 1 mol of glycerol-polyglycerol mixture of an average molecular weight between 140 and 210 with

a) 0.5 to 1.1 mol of a saturated aliphatic monocarboxylic acid with 6 to 10 C-atoms or mixtures thereof,

b) 0.5 to 1.1 mol of a saturated aliphatic monocarboxylic acid with 16 to 22 C-atoms or mixtures thereof,

c) 0.0 to 0.6 mol of an aliphatic saturated branched-chain monocarboxylic acid with 16 to 18 C-atoms or mixtures thereof,

d) 0.0 to 0.5 mol of a hydroxyl group-containing saturated monocarboxylic acid with 16 to 20 C-atoms or mixtures thereof and

e) 0.5 to 1.0 mol of saturated aliphatic dicarboxylic acids with 4 to 10 C-atoms or mixtures thereof, with a hydroxyl number of 20 to 100, an acid number and iodine number below 5.

2. Process for the production of wool wax substitutes based on homogeneous ester mixtures, characterised in that 1 mol of a glycerol-polyglycerol mixture of an average molecular weight between 140 and 210 is esterified with 0.5 to 1.1 mol of a saturated aliphatic monocarboxylic acid with 6 to 10 C-atoms or mixtures thereof, 0.5 to 1.1 mol of a saturated aliphatic monocarboxylic acid with 16 to 22 C-atoms or mixtures thereof, 0.0 to 0.6 mol of an aliphatic saturated branched-chain monocarboxylic acid with 16 to 18 C-atoms or mixtures thereof and 0.6 to 0.5 mol of a hydroxyl group-containing saturated monocarboxylic acid with 16 to 20 C-atoms or mixtures thereof at 200 to 250° C, preferably under vacuum to hydroxyl-containing partial esters, until an acid number below 50, preferably below 10, is achieved, and then is further esterified at 200 to 250° C, preferably under vacuum, with 0.5 to 1.0 mol of an aliphatic saturated carboxylic acid with 4 to 10 C-atoms or mixtures thereof until a hydroxyl number of 20 to 100 and an acid number below 5, preferably below 1, is achieved, and the crude product is deodourised in the usual manner.

## Claim for the designated state: AT

Process for the production of wool wax substitutes based on homogeneous ester mixtures, characterised in that 1 mol of a glycerol-polyglycerol mixture of an average molecular weight between 140 and 210 is esterified with 0.5 to 1.1 mol of a saturated aliphatic monocarboxylic acid with 6 to 10 C-atoms or mixtures thereof, 0.5 to 1.1 mol of a saturated aliphatic monocarboxylic acid with 16 to 22 C-atoms or mixtures thereof, 0.0 to 0.6 mol of an aliphatic saturated branched-chain monocarboxylic acid with 16 to 18 C-atoms or mixtures thereof and 0.0 to 0.5 mol of a hydroxyl group-containing saturated monocarboxylic acid with 16 to 20 C-atoms or mixtures thereof at 200 to 250° C, preferably under vacuum to hydroxyl-containing partial esters, until an acid number below 50, preferably below 10, is achieved, and then is further esterified at 200 to 250° C, preferably under vacuum, with 0.5 to 1.0 mol of an aliphatic saturated carboxylic acid with 4 to 10 C-atoms or mixtures thereof until a hydroxyl number of 20 to 100 and an acid number below 5, preferably below 1, is achieved, and the crude product is deodourised in the usual manner.

## Revendications pour les Etats contractants: BE, CH, DE, FR, GB, IT, LI, LU, NL, SE

1. Produits de remplacement pour la graisse de laine à base de mélanges d'esters homogènes, caractérisés par des produits d'estérification de 1 mole de mélange glycérine-polyglycérine d'un poids moléculaire moyen compris entre 140 et 210, avec

a) 0,5 à 1,1 mole d'un acide monocarboxylique aliphatique saturé ayant 6 à 10 atomes de C ou ses mélanges,

b) 0,5 à 1,1 mole d'un acide monocarboxylique aliphatique saturé ayant 16 à 22 atomes de C ou ses mélanges,

c) 0,0 à 0,6 mole d'un acide monocarboxylique aliphatique saturé, ramifié ayant 16 à 18 atomes de C ou ses mélanges,

d) 0,0 à 0,5 mole d'un acide monocarboxylique saturé hydroxylé ayant 16 à 20 atomes de C ou ses mélanges et

e) 0,5 à 1 mole d'acide dicarboxylique aliphatique saturé ayant 4 à 10 atomes de C ou ses mélanges, possédant un indice d'hydroxyle de 20 à 100, un indice d'acide et un indice d'iode inférieur à 5.

2. Procédé pour la préparation de produits de substitution de la graisse de laine à base de mélanges homogènes d'esters, caractérisé en ce qu'on estérifie en des esters partiels hydroxylés, entre 200 et 250° C, de préférence sous vide, 1 mole d'un mélange glycérine-polyglycérine possédant un poids moléculaire moyen compris entre 140 et 210 avec 0,5 à 1,1 mole d'un acide monocarboxylique aliphatique saturé ayant 6 à 10 atomes de C ou ses mélanges, 0,5 à 1,1 mole d'un acide monocarboxylique aliphatique saturé renfermant 16 à 22 atomes de C ou ses mélanges, 0,0 à 0,6 mole d'un acide monocarboxylique aliphatique saturé ramifié ayant 16 à 18 atomes de C ou ses mélanges et 0,0 à 0,5 mole d'un acide monocarboxylique hydroxylé saturé ayant 16 à 20 atomes de C ou ses mélanges, jusqu'à obtention d'un indice d'acide inférieur à 50, de préférence inférieur à 10, qu'on poursuit ensuite l'estérification entre 200 et 250°C, de préférence sous vide, avec 0,5 à 1 mole d'un acide dicarboxylique aliphatique saturé ayant 4 à 10 atomes de C ou ses mélanges jusqu'à obtention d'un indice d'hydroxyle de 20 à 100 et un indice d'acide inférieur à 5, de préférence inférieur à 1 et qu'on désodorise le produit brut selon une méthode connue en soi.

## Revendication pour l'Etat contractant: AT

Procédé pour la préparation de produits de substitution de la graisse de laine à base de mélanges homogènes d'esters, caractérisé en ce qu'on estérifie en des esters partiels hydroxylés, entre 200 et 250°C, de préférence sous vide 1 mole d'un mélange glycérine-polyglycérine possédant un poids moléculaire moyen compris entre 140 et 210 ayant 0,5 à 1,1 mole d'un acide monocarboxylique aliphatique saturé comptant 6 à 10 atomes de C ou ses mélanges, 0,5 à 1,1 mole d'un acide monocarboxylique aliphatique saturé renfermant 16 à 22 atomes de C ou ses mélanges, 0,0 à 0,6 mole d'un acide monocarboxylique aliphatique saturé ramifié ayant 16 à 18 atomes de C ou ses mélanges et 0,0 à 0,5 mole d'un acide monocarboxylique hydroxylé saturé ayant 16 à 20 atomes de C ou ses mélanges, jusqu'à obtenion d'un indice d'acide inférieur à 50, de préférence inférieur à 10, qu'on poursuit ensuite l'estérification entre 200 et 250°C, de préférence sous vide, avec 0,5 à 1 mole d'un acide dicarboxylique aliphatique saturé ayant 4 à 10 atomes de C ou ses mélanges jusqu'à obtention d'un indice d'hydroxyle de 20 à 100 et un indice d'acide inférieur à 5, de préférence inférieur à 1 et qu'on désodorise le produit brut selon une méthode connue en soi.